(19) Europäisches Patentamt
European Patent Office
Office européen des brevets

(11) Veröffentlichungsnummer: **0 139 996**
**B1**

(12) # EUROPÄISCHE PATENTSCHRIFT

(45) Veröffentlichungstag der Patentschrift: **04.11.87**

(21) Anmeldenummer: **84110152.0**

(22) Anmeldetag: **25.08.84**

(51) Int. Cl.⁴: **C 07 C 121/46**, C 07 C 121/66, C 07 C 121/75, C 07 C 19/02, C 07 C 21/24, C 07 D 237/06, C 07 D 239/24, C 09 K 19/08

(54) **Anisotrope Verbindungen und Flüssigkristallmischungen.**

(30) Priorität: **10.09.83 DE 3332692**

(43) Veröffentlichungstag der Anmeldung: **08.05.85 Patentblatt 85/19**

(45) Bekanntmachung des Hinweises auf die Patenterteilung: **04.11.87 Patentblatt 87/45**

(84) Benannte Vertragsstaaten: **CH DE FR GB LI**

(56) Entgegenhaltungen:
**EP-A-0 014 885**
**EP-A-0 134 570**
**CH-A-619 485**
**DE-A-2 439 703**
**DE-A-2 752 975**
**DE-A-3 148 148**
**DE-A-3 228 350**
**GB-A-2 111 993**
**US-A-4 311 610**
**US-A-4 330 426**

(73) Patentinhaber: **Merck Patent Gesellschaft mit beschränkter Haftung, Frankfurter Strasse 250, D-6100 Darmstadt (DE)**

(72) Erfinder: **Huynh- Ba, Tuong, Dr., 47 Chemin des Osches, CH- 1009 Pully (CH)**
Erfinder: **Osman, Maged A., Dr., Lerchenrain 1, CH- 8046 Zürich (CH)**

**EP 0 139 996 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue anisotrope Verbindungen zur Verwendung in Flüssigkristallmischungen (FK-Mischungen) für elektrooptische Anzeigen. Die Erfindung betrifft auch FK-Mischungen, welche die neuen anisotropen Verbindungen enthalten.

Für den Betrieb von elektrooptischen Anzeigen der verschiedenen Typen werden bekanntlich anisotrope Verbindungen benötigt, deren Moleküle zur Polarisierung in Richtung der Moleküllängsachse oder/und quer dazu bestimmte Substituenten tragen, welche diese Polarisierung bewirken. Der dadurch bewirkte Polarisierungseffekt äussert sich in der positiven oder negativen Anisotropie der Dielektrizitätskonstanten (DKA oder $\Delta\varepsilon$), die parallel ($\varepsilon_{\parallel}$) und senkrecht ($\varepsilon_{\perp}$) zur Molekülachse gemessen werden, wobei $\Delta\varepsilon = \varepsilon_{\parallel} - \varepsilon_{\perp}$.

Praktisch alle bekannten anisotropen Verbindungen fur Flussigkristallmischungen enthalten zwei bis vier cyclische Reste, die durch Brückenglieder (Kovalenzbindungen oder bestimmte zweiwertige Gruppen) miteinander verknüpft sind und meist auch sogenannte Flügelgruppen nach dem allgemeinen Schema

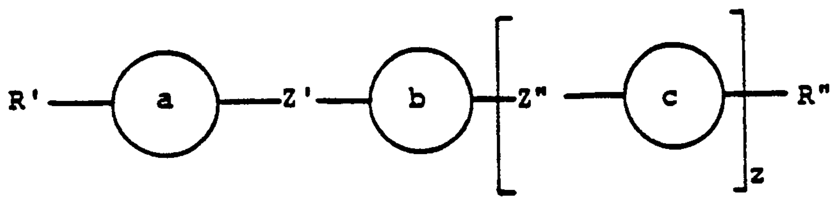

(I)

tragen, in welchem R', R" die Flügelgruppen darstellen, a, b und c die cyclischen Reste sind und Z', Z" die Brückenglieder bedeuten; z ist 0, 1 oder 2

Im typischen Fall wird die Polarisierung in Richtung der Moleküllängsachse - im folgenden kurz Längspolarisierung genannt - und dementsprechend der Beitrag zu $\varepsilon_{\parallel}$ durch eine stark polarisierende endständige Gruppe, wie die Cyanogruppe, am einen Molekülende (als R' oder R") und eine Alkyl- oder Alkoxygruppe am anderen Molekülende (R" oder R') bewirkt.

Die Polarisierung quer zur Moleküllängsachse - im folgenden kurz Querpolarisierung genannt - erfolgt allgemein durch polarisierende Substituenten in lateraler Position, und zwar bisher praktisch immer durch ringständige Substituenten, wie Cyano oder Halogenatome, meist Fluor oder Chlor, an den aromatischen cyclischen Resten, meist Benzolringen, nach dem Schema II

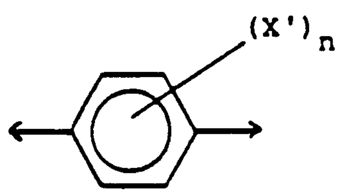

(II)

in der X' die polarisierende Gruppe ist, n 1 oder 2 bedeutet und die Pfeile annahernd der Moleküllängsachse entsprechen.

Aus dem Schema II ergibt sich ohne weiteres, dass diese übliche Art der Querpolarisation mit dem Ziel der Erhöhung von $\varepsilon_{\perp}$, z. B. für anisotrope Substanzen mit insgesamt negativer DKA oder für anisotrope Substanzen mit positiver DKA und gleichzeitig möglichst kleinen Werten des Verhältnisses $\frac{\Delta\varepsilon}{\varepsilon_{\perp}}$, stets zwangsläufig zu einer erheblichen Verbreiterung des Moleküls führt, die nachteilig ist, weil sie zu einer Erniedrigung des Klarpunktes führt und weitere Nachteile zur Folge haben kann. Auch die Beschränkung der Querpolarisation nach Schema II auf das Vorhandensein mindestens eines aromatischen Rings im Molekül kann nachteilig wirken.

Die Wünschbarkeit von anisotropen Verbindungen mit positiver DKA und möglichst geringen Werten von $\frac{\Delta\varepsilon}{\varepsilon_{\perp}}$ ist z. B. in der EP-A-0 019 665 erläutert und wird dort durch Kombination von Längs- und Querpolarisation mit ringständigen Substituenten erzielt.

Aber auch bei Längspolarisation ohne Querpolarisation des Moleküls ergeben sich Probleme dann, wenn ein stark polarisierender Substituent an einem cycloaliphatischen Rest hangen muss. Bekanntlich bietet der Ersatz der aromatischen Ringe durch cycloaliphatische Reste verschiedene Vorteile, wie niedrigere Viskosität und verminderte optische Anisotropie; es zeigt sich aber, dass ringstandige Substituenten, wie Cyanogruppen, an cycloaliphatischen Resten zu einer Erniedrigung des Klarpunktes führen oder sogar ein Verschwinden der Mesophase bewirken können, insbesondere wenn am cycloaliphatischen Rest gleichzeitig O oder N direkt gebunden ist. So zeigen z. B. Cyclohexylderivate mit endstandiger Nitrilgruppe gemäss Formel III

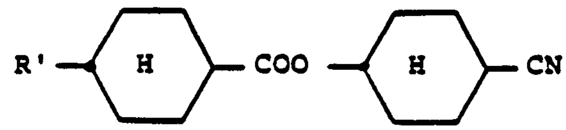

(III)

keine Mesophasen, während die analogen Phenylcyclohexylderivate der Formel IV

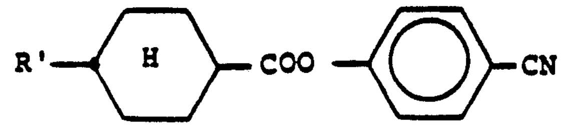

(IV)

noch Klärpunkte zwischen 70° und 80°C haben.

In den zur vorliegenden Erfindung führenden Untersuchungen wurde gefunden, dass die Konformationsenergie der das anisotrope Molekül bildenden Gruppierungen, insbesondere der polarisierenden Substituenten, bzw. die Differenz der freien Konformationsenergie $-\Delta G°_x$ der Gruppierungen bzw. Substituenten für deren Wirkung auf die Klarpunktserniedrigungen bedeutsam ist und dass sich dieser Effekt durch geeignete Wahl der Substituenten für Längs- oder/und Querpolarisierung vorteilhaft beeinflussen lassen sollte.

Aufgabe der Erfindung ist es, neue anisotrope Verbindungen für FK-Mischungen zum Betrieb von elektrooptischen Anzeigen anzugeben, welche die erwahnten probleme der Längspolarisierung oder/und der Querpolarisierung zu lösen gestatten.

Es wurde gefunden, dass diese Aufgabe durch Einführung eines stark polarisierenden Substituenten gewählt aus Cyano und Halogen, wie Fluor oder Chlor in den Alkylteil einer Flügelgruppe bestimmter anisotroper Moleküle gelöst werden kann.

Anisotrope Verbindungen vom Phenylbenzoat-Typ mit einer Flügelgruppe der Formel $-(CH_2)_m-CN$ (m = 0 bis 4) sind zwar bereits in der Patentschrift CH-A5-619 485 beschrieben, die durch die Konformationsenergie der das anisotrope Molekül bildenden Gruppierungen bedingten Vorteile der erfindungsgemäßen Verbindungen ist jedoch in diesen Verbindungen, die ausschließlich aromatische Ringe enthalten, praktisch bedeutungslos. Aus GB-21 11 993-A sind ferner anisotrope Acetonitrile mit einer Flügelgruppe der Formel $-CH_2CN$ bekannt, deren Mesobereiche im Vergleich zu den erfindungsgemäßen Eigenschaften (vgl. Tabelle I) drastisch eingeengt sind. Ein Vergleich von Verbindung 10 aus Tabelle II mit der strukturell ähnlichsten Verbindung aus GB-21 11 993-A, d. h. mit 4-(trans-4-Pentylcyclohexyl)-phenyl-acetonitril (Beispiel 31) zeigt, daß Verbindung 10 einen um ca. 47°C höheren Klärpunkt aufweist.

Gegenstand der Erfindung sind neue anisotrope Verbindungen der Formel (1)

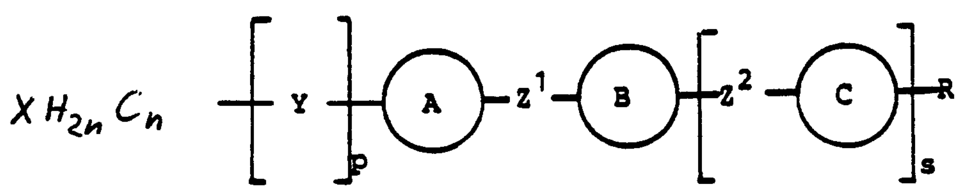

(1)

die wie folgt definiert ist: Die Ringe A, B und C sind gleich oder verschieden und gewählt aus cycloaliphatischen Resten der Formeln (1a) und (1b)

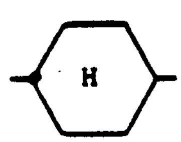

(1a)

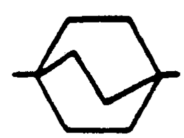

(1b)

d. h. die trans-1,4-Cyclohexyl- oder 1,4-Bicyclo-(2,2,2)-octyl-Reste, und aromatischen Resten der Formeln (1c), (1d), (1e)

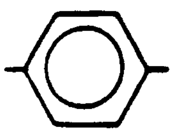

(1c)

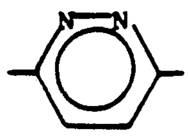

(1d)

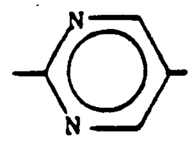

(1e)

d. h. die 1,4-Phenyl-, 3,6-Pyridazinyl- oder 2,5-Pyrimidin-Reste. Der Rest X, d. h. die im Molekül vorhandene stark polarisierende Gruppe, ist die Cyanogruppe oder ein Halogenatom, wobei als Halogenatome Fluor und Chlor gegenüber Brom bevorzugt werden und Jod am wenigsten bevorzugt ist.

Y ist eine fakultative Strukturkomponente (d. h. p ist 0 oder 1), die das Sauerstoffatom (-O-), die Carboxylgruppe (-C(O)-O- oder -O(O)C-) oder die Iminogruppe (-N(H)-) bedeutet. Wenn A einen aliphatischen Ring der Formeln (1a) oder (1b) bedeutet, ist p vorzugsweise 0.

Die Brücken $Z^1$ und $Z^2$ können gleich oder verschieden sein und Kovalenzbindungen oder Gruppen der Formeln -COO-, $-CH_2O-$ oder

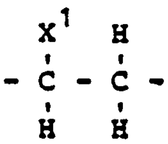

bedeuten, wobei $X^1$ Wasserstoff ist oder die für X angegebene Bedeutung hat und die Gruppen auch in der jeweils umgekehrten Folge -OOC-, $-OCH_2-$ und $-CH_2C(X^1)(H)-$ stehen können, soweit nicht durch die unten genannten Massgaben ausgeschlossen.

Wenn $X^1$ die für X angegebene Bedeutung hat, d. h. Cyano oder Halogen (F und Cl als Halogen bevorzugt) ist, trägt das entsprechende Brückenglied sur Querpolarisierung des Moleküls der Formel (1) bei. $X^1$ kann aber nur dann X bedeuten, wenn auch die Flügelgruppe R bzw. $R^1$ gleich Halogen oder Cyano ist.

Der Index n bedeutet eine Zahl von 1 bis 12.

Der Ring C ist eine fakultative Komponente erfindungsgemässer Verbindungen, d. h. s ist ebenso wie p 0 oder 1.

R kann die für $X^1$ angegebene Bedeutung haben, wenn der benachbarte Ring C (wenn s = 1) bzw. B (wenn s = 0) ein aromatischer Rest der Formeln (1c), (1d) oder (1e) ist; alternativ ist R eine Alkyl- ($H_{2m+1}C_m$-), Alkoxy- ($H_{2m+1}C_m$-O-), Alkoxycarbonyl- ($H_{2m+1}C_m$-OC(O)-), Alkylcarbonyloxy-($H_{2m+1}C_m$-C(O)-O-) oder Alkylaminogruppe ($H_{2m+1}C$-N(H)-), deren Alkylteil 1 bis 12 C-Atome (m = 1-12) in gerader oder verzweigter und gegebenenfalls chiraler Kette enthält. Beispiele für die Alkylteile der genannten Gruppen sind Methyl-, Ethyl-, Propyl-, Butyl-, Pentyl-, Hexyl-, Heptyl-, Octyl-, Nonyl-, Decyl-, Undecyl- und Dodecylgruppen, einschliesslich der isomeren bzw. chiralisomeren Alkyle, wie 1-Methylpropyl-, 1-Methylbutyl-, 2-Methylbutyl-, 1-Methylpentyl-, 2-Methylpentyl, 3-Methylpentyl-, 1- oder 2- oder 3- oder 4-Methylhexyl, 1- oder 2- oder 3- oder 4- oder 5-Methylheptyl sowie die nach diesem Prinzip gebildeten weiteren Alkyl-Alkylgruppen mit asymmetrischen C-Atom.

Die Alkylteile von R können einen oder mehrere Substituenten, und zwar Halogenatome oder Cyanogruppen tragen, wobei aber an jeweils einem C-Atom des Alkylteils höchstens jeweils ein derartiger Substituent hängt. Weiterhin kann R einen Rest der Formel (1f)

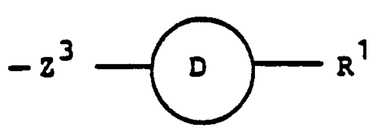

(1f)

bedeuten, in welcher der Ring D die für die Ringe A, B und C angegebene Bedeutung, $Z^3$ die für $Z^1$ und $Z^2$ angegebene Bedeutung und $R^1$ die für R angegebene Bedeutung mit Ausnahme des Restes der Formel (1f) hat.

Die Formel (1) unterliegt erfindungsgemäss folgenden beschränkenden Massgaben (a), (b), (c), (d), und (e):

(a) an keinen der im Molekül der Formel (1) vorhandenen cycloaliphatischen Reste der Formeln (1a) oder (1b) ist gleichseitig direkt ein Sauerstoffatom einerseits und direkt ein Sauerstoff- oder Stickstoffatom oder ein Rest Cyano oder Halogen andererseits gebunden; das Molekül darf mit anderen Worten keine Ringe der Formeln

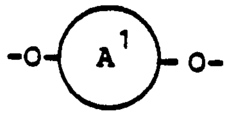

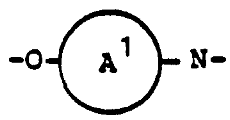

und

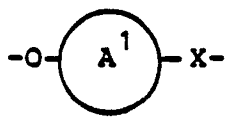

enthalten, wobei $A^1$ ein Rest der Formeln (1a) oder (1b) ist;

b) an keinen der im Molekül der Formel (1) vorhandenen aromatischen Reste der Formeln (1c), (1d) oder (1e) sind Gruppen der Formeln $-CH_2O-$ oder $-C(X)(H)-$ direkt mit den C-Atomen dieser Gruppen gebunden ausser wenn X das Fluoratom ist; das Molekül darf mit anderen Worten keine Ringe der Formeln

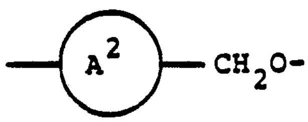

und

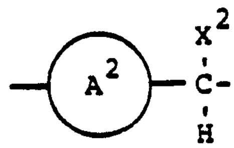

enthalten, wobei $A^2$ ein Rest der Formeln (1c), (1e) und $X^2$ Cyano, Chlor, Brom oder Jod aber nicht Fluor ist;

(c) der Rest $XH_{2n}C_n-$ eine Gruppe der Formel $NC-CH_2CH_2-$bedeutet, falls die Substitution durch X endständig ist,

(d) das Molekül die Formel $NC-CH_2CH_2-$ 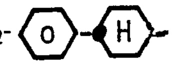

$C_{1-12}$-Alkyl hat, wenn im Molekül der Formel (1) der Ring A einen aromatischen Rest gebunden an eine $-CH_2CH_2CN$-Gruppe bedeutet, und

(e) $X^1$ nur dann X sein kann, wenn R bzw. $R^1$ gleich Halagen oder Cyano ist.

Die Massgaben (a), (b) und (c) sind dadurch bedingt, dass Verbindungen der Formel (1) ohne diese Massgaben keine oder zu tiefe Klärpunkte besitzen bzw. nicht die erforderliche Stabilität haben. Die Massgaben (d) und (e) dienen der Abgrenzung.

Neu und überraschend ist die der Erfindung zugrundliegende Erkenntnis, dass die Einführung von polarisierenden Substituenten in flügelständige Alkylgruppen eine Vielzahl von neuen anisotropen Verbindungen mit niedirgen Werten $\frac{\Delta\varepsilon}{\varepsilon_{\perp}}$ bietet, und zwar unabhängig davon, ob die Ringe des Moleküls nur cyclophatische Reste oder nur aromatische Reste sind, oder ob cycloaliphatische Reste kombiniert mit aromatischen Resten vorliegen. In diesem Fall enthält das Molekül der neuen erfindungsgemässen Verbindungen (1) eine Kombination von längspolarisierendem X und querpolarisierendem X, z. B. ein endständiges X am Ende einer Flügelgruppe oder direkt an einem aromatischen Ring zusammen mit mindestens einem lateralen X als Teil einer Flügelgruppe.

Ferner bietet die der Erfindung zugrundeliegende Erkenntnis eine Vielzahl von neuen, stark längspolarisierten anisotropen Verbindungen, bei welchen das längspolarisierende Cyano oder Halogenatom endständig an einem Alkylteil einer Flügelgruppe ist, die an einem cycloaliphatischen Rest hängt; dadurch sind längspolarisierte Verbindungen mit hohen Klärpunkten erhältlich.

Diese Längspolarisierung durch Cyano oder Halogen in der Flügelgruppe eines cycloaliphatischen Restes kann gegebenenfalls mit querpolarisierendem Cyano oder Halogen in einer Flügelgruppe oder/und einem Brückenglied kombiniert sein.

Wenn die erfindungsgemässe Verbindung (1) eine ausgeprägt positive DKA aufweisen soll, wird sie meist eine Flügelgruppe tragen, die ein endständiges X - vorzugsweise Cyano - enthält oder daraus besteht. Hierbei ist, wie auch in den nachberücksichtigen, dass auch ein nicht-endständiges X in den Flügelgruppen einen Beitrag zur Längspolarisierung liefern kann.

Wenn die Verbindung (1) einen möglichst kleinen Wert von $\frac{\Delta\varepsilon}{\varepsilon_{\perp}}$ bei mehr oder weniger ausgeprägt positiver DKA haben soll, trägt sie eine Flügelgruppe, die ein endständiges X, vorzugsweise Cyano, enthält oder daraus besteht, kombiniert mit mindestens einem nicht-endständigen X in einer Flügelgruppe und/oder einem brückenständigen X.

Verbindungen (1) mit negativer DKA werden erhalten, wenn die Summe der lateralen Polarisierungsbeiträge von X in den Flügeln oder/und Brücken die Summe der längspolarisierenden Beiträge übersteigt bzw. wenn die längspolarisierenden Gruppen fehlen.

Bevorzugte Gruppen erfindungsgemässer Verbindungen der Formel (1) sind in den Ansprüchen 2-13 definiert und haben insbesondere folgende Merkmale, einzeln oder in Kombination:

- Das Molekül enthält zwei oder höchstens drei cyclische Reste und insgesamt 1 bis höchstens 4 Cyano- oder Halogenreste;
- der Ring A ist vorzugsweise ein cycloaliphatischer Rest, insbesondere ein solcher der Formel (1a);
- der Ring A trägt vorzugsweise eine $XC_{1-7}$-Alkylgruppe, insbesondere Cyano-$C_{1-7}$-Alkylgruppe;
- wenn X Halogen ist, wird Fluor und Chlor bevorzugt;
- wenn R bzw. $R^1$ einen substituierten Alkylteil enthält, trägt dieser vorzugsweise einen oder zwei Substituenten, wobei als Halogenatome Fluor und Chlor bevorzugt sind;
- das Molekül der Formel (1) enthält vorzugsweise insgesamt höchstens eine Carboxylgruppe -COO- oder -OOC-;
- das Molekül der Formel (1) enthält vorzugsweise höchstens einen aromatischen Ring der Formeln (1c), (1d), (1e);
- das Molekül der Formel (1) enthält mindestens ein Brückenglied Z in Form der Kovalenzhindung;
- das Molekül der Formel (1) enhält zwei oder drei cycloaliphatische Reste der Formeln (1a), (1b);
- wenn R bzw. $R^1$ einen Alkylteil besitzt, enthält dieser vorzugsweise 3 bis 9 C-Atome;
- wenn R bzw. $R^1$ Wasserstoff oder X bedeutet, ist der benachbarte Ring ein aromatischer Rest der Formeln (1c), (1d) oder (1e);
- der Rest X in der linken Flügelgruppe und der gegebenenfalls vorhandene Substituent des Alkylteils von R bzw. $R^1$ hängt vorzugsweise an einem C-Atom der Alkylkette, welches vom zugehörigen Ring durch höchstens 2 C-Atome entfernt ist.

Die folgenden Formeln erläutern die Struktur spezieller Gruppen von erfindungsgemässen Verbindungen, wobei $A^1$, $B^1$, $C^1$ jewells die cyloaliphatischen Reste (1a), (1b) und $A^2$, $B^2$, $C^2$ jeweils die aromatischen Reste (1c), (1d), (1e) sind und $R^4$ $C_{1-12}$-Alkyl, $C_{1-12}$-Alkoxy, $XC_{1-7}$-Alkyl, Cyano, F oder Cl und $R^5$ $C_{1-12}$-Alkyl, $C_{1-12}$-Alkoxy, $C_{1-12}$-Alkoxycarbonyl oder $C_{1-12}$-Alkylcarbonyloxy bedeutet; die Zeichen A, B, C, $Z^1$, $Z^2$ sowie $R^1$ haben die oben angegebene Bedeutung. Vorzugsweise sind $R^4$ und $R^5$ $C_{1-12}$-Alkyl.

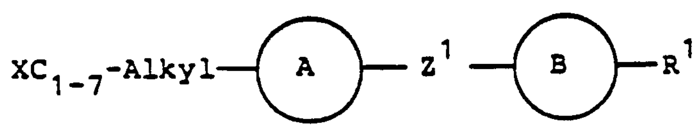

(10)

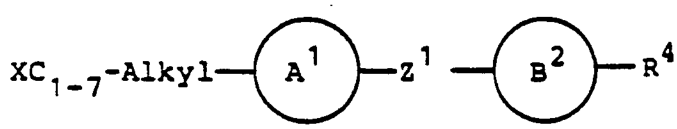

(11)

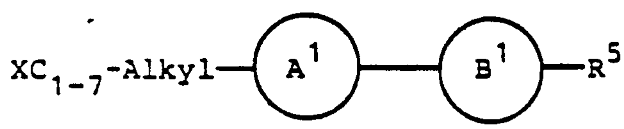

(12)

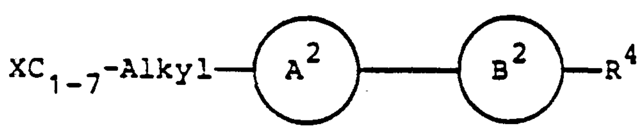

(13)

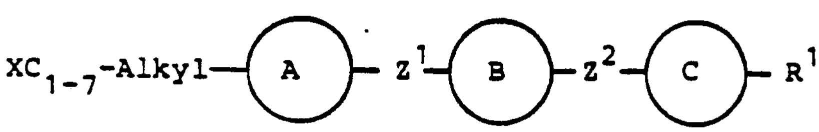

(14)

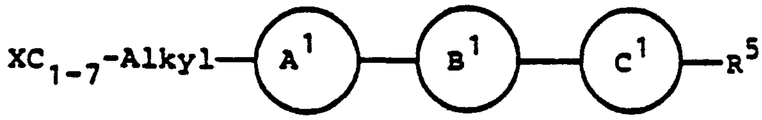

(15)

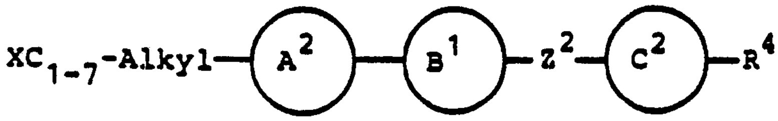

(16)

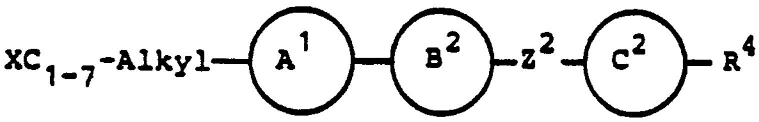

(17)

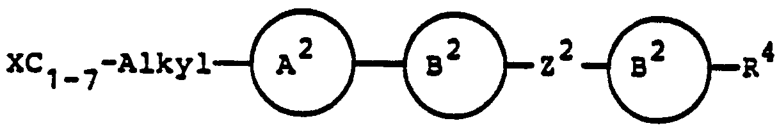

(18)

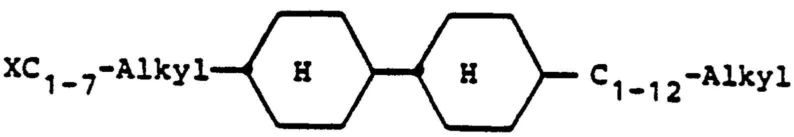

(19)

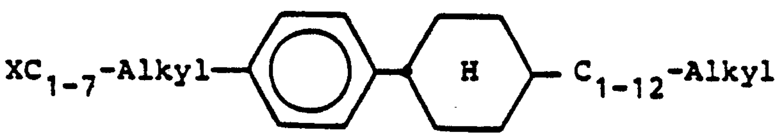

(20)

$XC_{1-7}$-Alkyl bedeutet eine durch X substituierte n-Alkylgruppe mit 1-7 C-Atomen. Die substitution ist vorzugsweise endständig in ω-Postition. $XC_{1-7}$-Alkyl ist insbesondere bevorzugt $NC-CH_2CH_2$-.

Erfindungsgemäss sind ferner Flüssigkristallmischungen, die mindestens eine verbindung der Formel (1) enthalten, z. B. in Anteilen von 1-30 Mol%, wobei die Mischung auch mehrere verschiedene verbindungen der Formel (1), z. B. in eine, Anteil von bis insgesamt 90 Mol%, enthalten kann. Als weitere Komponenten können die erfindungsgemässen FK-Mischungen bekannte anisotrope verbindungen sowie die dem Verwendungszweck entsprechenden Zusätze, wie Farbstoffe, insbesondere pleochroitische Farbstoffe, optisch aktive bzw. cholesterische Komponenten und dergleichen enthalten.

Die neuen verbindungen der Formel (1) sind nach verschiedenen an sich bevannten Methoden erhältlich; eine erste allgemeine Methode beruht beispielsweise darauf, dass man eine dem Molekül (1) entsprechende Vorverbindung ohne X-Rest bzw. ohne X-Alkyl durch Einführung von X bzw, X-Alkyl modifiziert, z. B. durch Halogenieren, wie Bromieren, gegebenenfalls Transhalogenieren und gegebenenfalls Nitrilieren oder durch Friedel-Crafts-Reaktion und Reduktion in an sich bekannter Weise.

Eine zweite allgemeine Methode beruht z. B. darauf, dass man das Molekül (1) durch Vereinigen von entsprechenden Molekülfragmenten bildet, z. B. durch Kondensation, Veresterung oder Verätherung an einem Brückenglied.

Entsprechende Vorverbindungen bzw. Molekülfragmente sind entweder als solche bekannt oder können analog wie die bekannten Verbindungen erhalten werden.

Ein allgemeines Beispiel für die erste Methode ist im folgenden Schema verläutert; durch Wiederholungen der Schritte: Bromierung/Carboxylierung/Reduktion/Bromierung kann die Kettenlänge der X-tragenden Flügelgruppe weiter vergrössert werden, wobei der Rest X jeweils endständig ist; die Bildung eine, Alkylflügelgruppe mit nicht-endständigem X, z. B. Cyano, ist in Schema Va erläutert, in welchem jeweils von einer "Verbindung (1)", d. h. einer Verbindung der Formel (1), bzw. einer entsprechenden "Vorverbindung" ausgegangen werden kann.

Das Schema Vb erläutert ein spezielles Beispiel zur Herstellung von geeigneten "Vorverbindungen-COOH", d. h. von entsprechenden Carbonsäuren für die Synthese gemäss Schema V oder zur Herstellung von Verbindungen der Formel (1) mit ringständigem terminalem Cyano durch Umwandlung der Carboxylgruppe nach bekannten Methoden, z. B. direkt oder über das entsprechende Amid. Dabei bedeutet "X-Alkyl" in Schema Vb eine Gruppe der Formel $XH_{2n'}$ $C_{n'}$- in der n' jeweils um Eins kleiner ist als das in Formel (1) gewünschte n.

**Schema V**

Vorverbindung -COOH

(Reduktion)

Vorverbindung $-CH_2OH$

(Bromierung)

Verbindung (1) $-CH_2Br$

(Nitrilierung)

Verbindung (1) $-CH_2CN$

(Carboxylierung; $-CH_2COOH$)

(Reduktion; $-CH_2CH_2OH$)

(Bromierung)

Verbindung (1) $-CH_2CH_2Br$

(Nitrilierung)

Verbindung (1) $-CH_2CH_2CN$

**Schema Va**

Vorverbindung -CN

oder

Verbindung (1) -CN

(1) R'MgBr

(2) TPSH/KCN

Verbindung (1) -CH(CN)-R'

**Schema Vb**

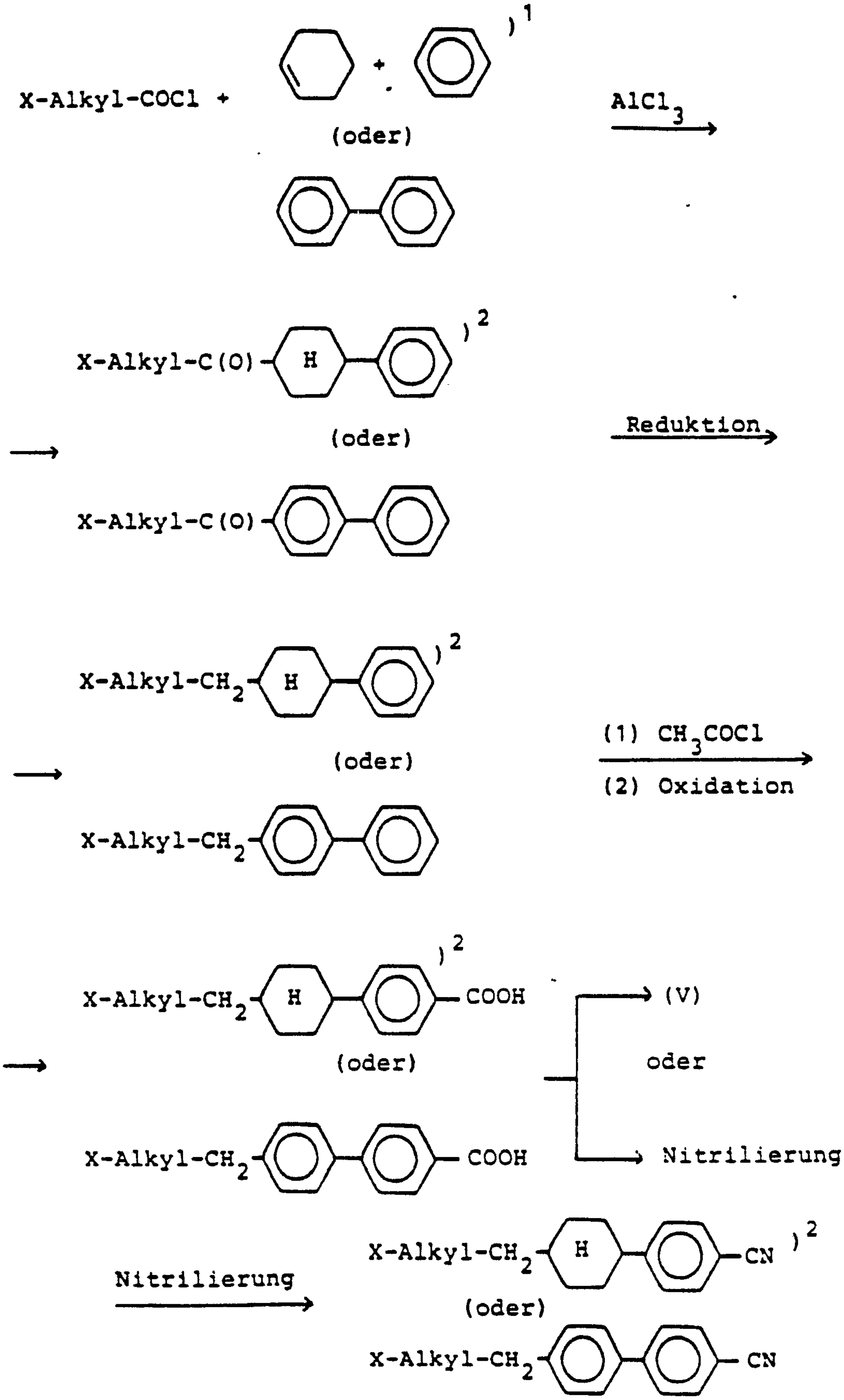


)[1] Siehe J.A.C.S. 67 (1945) 1045
)[2] gegebenenfalls nachfolgende Trennung und Gewinnung des trans-Isomeren

Die Reduktion der Carboxylverbindungen kann z. B. mit Lithiumaluminiumhydrid erfolgen; die Bromierung erfolgt meist zweckmässig mit elementarem Brom in Gegenwart von Triphenylphosphin, die Nitrilierung z. B. mit Kupfer(I)-cyanid oder KCN; zur Carboxylierung ist die Grignard-Methode oder unter Umständen die Nitrilgruppenhydrolyse geeignet.

Allgemeine Beispiele für bekannte oder in bekannter weise erhältliche Carboxyl-Vorverbindungen sind die folgenden, wobei $R^1$, B, C, Z und Z die oben angegebene Bedeutung haben:

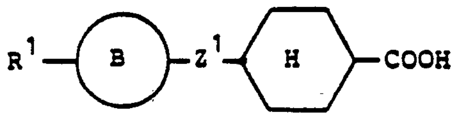
(V-1)

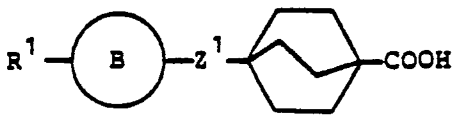
(V-2)

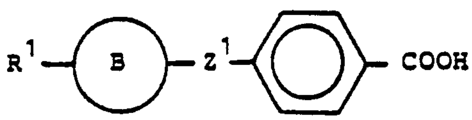
(V-3)

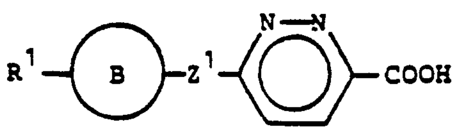
(V-4)

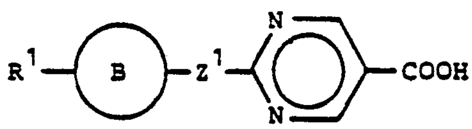
(V-5)

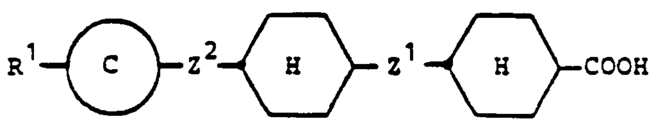
(V-6)

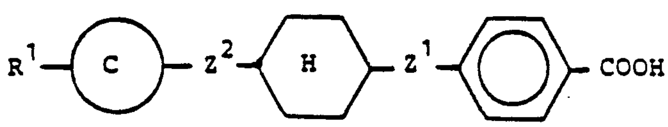
(V-7)

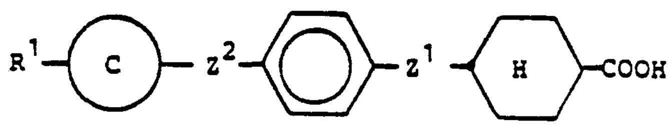
(V-8)

Ein allgemeines Beispiel für die zweite Methode zur Herstellung erfindungsgemässer Verbindungen (1) ist im folgenden Schema VI erläutert, in welchem die herzustellende Verbindung (1) aus zwei Fragmenten, z. B. $Fragment^a$ und $Fragment^b$, besteht, die durch ein Brückenglied Z, z. B. eine Carboxyloder Methoxygruppe miteinander verbunden sind. Je nach Art der Brückengruppe ist die Reaktion z. B. eine Veresterung, Verätherung oder dergleichen und das Fragment kann einen Teil ($Z^a$, $Z^b$) der zu bildenden Brücke tragen; $L^1$ und $L^2$ sind entsprechende Abgangsgruppen.

**Schema VI**

$Fragment^a Z^a L^1 + L^2 Z^b Fragment^b \rightarrow Fragment^a\text{-}Z\text{-}Fragment^b$

Auch für die Synthese nach Schema VI sind geeignete verbindungen entweder bekannt oder in an sich bekannter Weise erhältlich.

Zur weiteren Erläuterung der Erfindung dienen die folgenden Beispiele:

**Beispiel 1**

Herstellung von 4-trans-Cyanoethyl-cyclohexyl-trans-propyl-cyclohexyl-1-carboxylat

4-trans-Propyl-cyclohexan-1-carbonsäure (25 mM) wurde mit Thionylchlorid (40 ml) 30 min auf Rückflusstemperatur erwärmt. Das entstandene Säurechlorid wurde vom überschüssigen Thionylchlorid befreit. Das so gewonnene Säurechlorid wurde nun zu einer Lösung von 4-trans-Cyanoethyl-cyclohexanol (Schmelzpunkt 52°C, 25 mM, folgendermaßen erhältlich: 2-(4-Hydroxyphenyl)-propionamid wird in 95 %iger Essigsäure katalytisch hydriert (Nishimura-Katalysator). Das erhaltene 1:1 cis-trans-Gemisch wird mit Aluminiumopropylat ins Gleichgewicht gebracht. Der erhaltene trans-Alkohol wird acetyliert, mit $SOCl_2$ in das Nitril übergeführt und nachfolgend mit NaOH die Acetylgruppe hydrolysiert) in 100 ml Pyridin zugetropft. Nach Beendigung der Reaktion wurde das Gemisch in überschüssige verdünnte Salzsäure gegossen und mit Methylenchlorid extrahiert. Das aus dem Extrakt durch Eindampfen erhaltene Produkt wurde umkristallisiert. Die so erhaltene Zielverbindung dieses Beispiels ist monotrop flüssigkristallin, Schmelzpunkt 73,0°C, Klärpunkt 14,6°C.

**Beispiel 2**

Herstellung von 4-trans-Cyanoethyl-cyclohexyl-4-trans-pentyl-cyclohexyl-1-carboxylat

4-trans-Pentyl-cyclohexan-1-carbonsäure (36,5 mM) wurde mit Thionylchlorid (50 ml) 1 Std. unter Rückfluss gekocht. Das überschüssige Thionylchlorid wurde destilliert. Das entstandene Säurechlorid wurde zu einer Lösung von 36,5 mM 4-trans-Cyanoethyl-cyclohexanol in 100 ml Pyridin zugetropft. Die Reaktionsmischung wurde bis zur Beendigung der Reaktion gerührt und dann auf verdünnte Salzsäure gegossen. Das Produkt wurde mit Methylenchlorid extrahiert. Zur Reinigung wurde das Produkt umkristallisiert. Es zeigt eine monotrope flüssigkristalline Phase, Schmelzpunkt 61,5°C, Klärpunkt 39,3°C.

**Beispiele 3-7**

Nach dem im Reaktionsschema V bzw. Va angegebenen Verfahren wurden aus den entsprechenden Carbonsäuren der Formel (30)

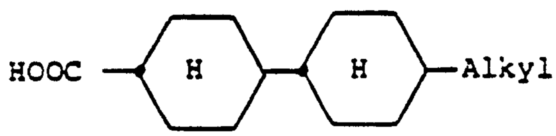

(30)

die erfindungsgemässen Verbindungen der Formel (31)

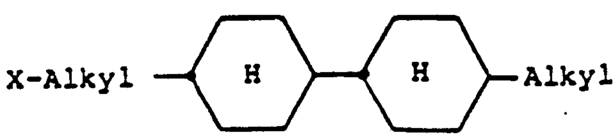

(31)

hergestellt und ihre Übergangstemperaturen bestimmt.

Die Ergebnisse sind in der folgenden Tabelle I zusammengestellt

**Tabelle I**

| Beispiel | X-Alkyl | Alkyl | Schmelzp. | Klärp. (°C) |
|---|---|---|---|---|
| 3 | $Br-CH_2-CH_2-$ | n-Pentyl | 46.0 | 67.9 |
| 4 | $NC-CH_2-CH_2-$ | n-Pentyl | 9 | 108.8 |
| 5 | $H_3C(CH_2)_3C(H)(CN)-$ | n-Pentyl | 35 | 80.0 |

**Beispiel 6**

Die Herstellung von 4-trans-Cyanoethyl-4'-trans-propyl-bicyclohexan gemäß Schema V;

Schritt 1: Eine Lösung von 4'-Propyl-trans,trans-bicyclo-hexan-4-carbonsäure (0,1M) [R. Eidenschink, D, Erdmann, J. Krause und L. Pohl, Angew. Chem. Int. Ed. 17, 133 (1978)] in trockenem THF wurde zu einer Suspension von $LiAlH_4$ (10 g) in 100 ml trockenem THF bei 0°C getropft. Das Reaktionsgemisch wurde eine Stunde unter Rückfluß gekocht, in kalte verdünnte Salzsäure gegossen und das Produkt mit Ether extrahiert.

Schritt 2: Brom (0,12 M) wurde zu einer Suspension von $P(Ph)_3$ (0,12 M) in trockene, $CH_3CN$ bei 0°C getropft. Zu dieser Suspension wurde eine Lösung des oben hergestellten Alkohols in trockenem $CH_3CN$ getropft und die Mischung 15 min bei dieser Temperatur gerührt, Dann wurde das Lösungsmittel abdestilliert und der Rückstand 30 min auf 130°C erhitzt. Nach üblicher Aufarbeitung des Reaktionsgemisches wurde das Rohprodukt mit $CH_2Cl_2$ extrahiert und die Lösung wurde zu Hexan gegeben, um das Triphenylphosphinoxid auszufällen. Das Produkt wurde durch Chromatographie (Kieselgel/Toluol) gereinigt.

Schritt 3: Eine Lösung des oben hergestellten Bromides in trockenem Ether wurde zu einer Suspension von Magnesium (4g) in trockenem Ether getropft und bei Raumtemperatur 1 Stunde gerührt. Anschließend wurde trockenes $CO_2$-Gas 30 min durch das Reaktionsgemisch geleitet, wonach es wie üblich aufgearbeitet wurde. Das Rohprodukt wurde aus Toluol oder Hexan umkristallisiert. Die Schritte 1 und 2 wurden dann wiederholt mit diesem Produkt als Ausgangsmaterial.

Schritt 4: Ein Gemisch des so erhaltenen 4-trans-Bromethyl-4'-trans-propylbicyclohexan mit festem KCN wurde 2 Stunden in DMSO auf 100°C erhitzt und anschließend in Wasser gegossen. Das Reaktionsprodukt wurde mit Ether extrahiert und über eine kurze Kieselgelsäule mit Toluol als Laufmittel filtriert. Es wurde aus EtOH umkristallisiert, Schmelzpunkt 13°C, Klärpunkt 99°C.

**Beispiele 7-8**

Nach dem Verfahren gemäss Reaktionsschema V bzw. Va wurden aus den entsprechenden Carbonsäuren der Formel (80)

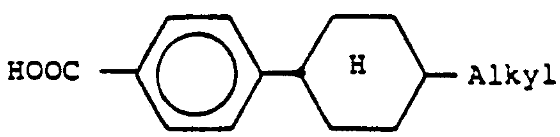

(80)

die entsprechenden erfindungsgemässen Verbindungen der Formel (81)

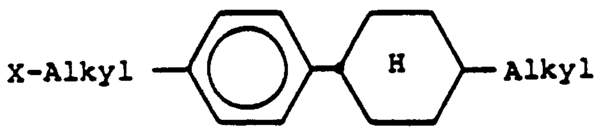

(81)

hergestellt. Die erhaltenen anisotropen Verbindungen sind in der folgenden Tabelle II zusammangestellt.

**Tabelle II**

| Beispiel | X-Alkyl | Alkyl | Schmelzp. | Klärp. (°C) |
|---|---|---|---|---|
| 7 | $NC-CH_2-CH_2-$ | n-Pentyl | 44.8 | (28.3) |
| 8 | $H_3C(CH_2)_3C(H)(CN)-$ | n-Pentyl | 34 | |

**Beispiel 9**

Herstellung von 4-Cyanoethylphenyl-4-trans-pentyl-cyclohexyl-1-carboxylat

Eine Lösung von trans-4-Pentylcyclohexancarbonsäurechlorid (0,1 M) in Pyridin wurde zu einer Lösung von 4-trans-Cyanoethyl-cyclohexanol (0,1 M) in Pyridin bei 0 - 5°C getropft und die Mischung bei dieser Temperatur 2 Stunden gerührt. Das Reaktionsgemisch wurde dann wie üblich aufgearbeitet und das Produkt zweimal aus Methanol umkristallisiert, Schmelzpunkt 75°, Klärpunkt 51°.

Weitere Beispiele von Verbindungen der Formel (1) sind die folgenden;

4-(2-Fluorobutyl)-4'-cyano-biphenyl
4-(1-Fluoropentyl)-4'-cyano-biphenyl
4-(2-Fluoropentyl)-4'-cyano-biphenyl
4-(3-Fluoropentyl)-4'-cyano-biphenyl
4-(2-Chloroethyl)-4''-(2-flouropropyl)-p-terphenyl

4-(1-Fluoropentyl)-4''-cyano-p-terphenyl
4-(2-Fluoropropyl)-4''-cyano-p-terphenyl
1-(4-trans-(1-Fluoropentyl)-cyclohexyl)-4-cyano-benzol
1-(4-trans-(2-Cyanoethyl)-cyclohexyl)-4-butoxy-benzol
1-(4-trans-(2-Fluoropentyl)-cyclohexyl)-4-cyano-benzol
1-(4-trans-(3-Fluoropentyl)-cyclohexyl)-4-cyano-benzol
1-(4-trans-(2-Cyanoethyl)-cyclohexyl)-2-(4-cyanophenyl)-ethan
3-(4-(3-Fluoropentyl)-phenyl)-6-(4-cyanophenyl)-pyridazin
3-(4-trans-(2-Cyanoethyl)-cyclohexyl)-6-(4-pentylphenyl)-pyridazin
3-(4-trans-(2-Cyanoethyl)-cyclohexyl)-6-butoxy-pyridazin
1-(4-trans-(2-Cyanoethyl)-cyclohexyl)-2-(4-(5-(2-chloroethyl)-2-pyrimidin)-phenyl)-ethan
1-(4-trans-(2-Cyanoethyl)-cyclohexyl)-2-(4-(5-(3-fluoropropyl)-2-pyrimidin)-phenyl)-ethan
4-trans-(2-Bromoethyl)-4'-trans-propyl-bicyclohexan
4-trans-(2-Cyanoethyl)-4'-trans-propyl-bicyclohexan
4-trans-(2-Cyanoethyl)-4'-trans-pentyl-bicyclohexan
4-trans-(2-Cyanoethyl)-4''-trans-(4-pentyl-1-bicyclo-(2,2,2)-octyl)-bicyclohexan
4-trans-(2-Cyanoethyl)-cyclohexyl-4-cyano-benzoat
4-trans-(2-Cyanoethyl)-cyclohexyl-4-pentyl-benzoat
1-(4-trans-(2-Cyanoethyl)-cyclohexyl)-methoxy-4-butoxy-benzol
4-trans-(2-Cyanoethyl)-cyclohexyl-4-trans-(4-butoxyphenyl)-cyclohexan-1-carboxylat
4-trans-(2-Cyanoethyl)-cyclohexyl-4-(4-trans-(2-cyanoethyl)-cyclohexyl)-benzoat
4-trans-(2-Cyanoethyl)-cyclohexyl-4-(4-trans-pentyl-cyclohexyl)-benzoat
1-(4-trans-(2-Cyanoethyl)-cyclohexyl)-4-(4-trans-(3-fluoropropyl)-cyclohexyl)-methoxy-benzol
4-trans-(Fluoromethyl)-cyclohexyl-4'-(2-cyanoethyl)-bicyclohexan-4-carboxylat
4-trans-(2-Cyanoethyl)-cyclohexyl-4'-(3-fluoropropyl)-bicyclohexan-4-carboxylat
1-(4'-(2-Cyanoethyl)-bicyclohex-4-yl)-methoxy-4-trans-(2-cyanoethyl)-cyclohexan
4-trans-(2-Cyanoethyl)-cyclohexyl-4-(2-cyanoethyl)-bicyclo-(2,2,2)-octan-1-carboxylat
1-(4-(2-Cyanoethyl)-1-bicyclo-(2,2,2)-octyl)-methoxy-4-trans-(2-cyanoethyl)-cyclohexan

Es folgen Beispiele für erfindungsgemäße Flüssigkristallgemische;

**Beispiel A**

Man stellt ein Flüssigkristallgemisch her bestehend aus
9 % 2-p-Cyanphenyl-5-propyl-1,3-dioxan
12 % 2-p-Cyanphenyl-5-butyl-1,3-dioxan
9 % 2-p-Cyanphenyl-5-pentyl-1,3-dioxan
6 % 2-p-Octoxyphenyl-5-pentyl-pyrimidin
5 % 2-p-Nonoxyphenyl-5-pentyl-pyrimidin
5 % 2-p-Heptoxyphenyl-5-hexyl-pyrimidin
4 % 2-p-Nonoxyphenyl-5-hexyl-pyrimidin
6 % 4,4'-Bis-(trans-4-propylcyclohexyl)-biphenyl
9 % 4-(trans-4-pentylcyclohexyl)-4'-(trans-4-propylcyclohexyl)-biphenyl
17 % r-1-Cyan-1-pentyl-cis-4-(trans-4-propylcyclohexyl)-cyclohexan
und
18 % 4-trans-Cyanoethyl-4'-trans-propyl-bicyclohexan.

**Beispiel B**

Man stellt ein Flüssigkristallgemisch her bestehend aus
27 % trans,trans-4-Pentylcyclohexylcyclohexan-4'-carbonitril
17 % trans,trans-4-Propylcyclohexylcyclohexan-4'-carbonitril
30 % 4-trans-Cyanoethyl-4'-trans-pentyl-bicyclohexan
16 % trans-4-Propylcyclohexancarbonsäure-(p-ethoxyphenyl-ester) und
10 % trans,trans-4-Pentyl-4'-butyryloxybicyclohexan.

**Patentansprüche**

1. Neue anisotrope Verbindungen sur Verwendung in Flüssigkristallmischungen für elektrooptische Anzeigen, gekennzeichnet durch die Formel (1)

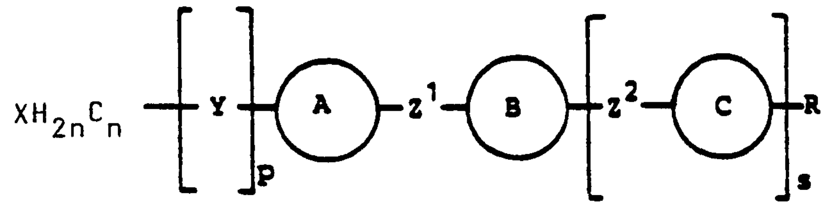 (1)

in welcher die Ringe A, B und C gleich oder verschieden und gewählt sind aus cycloaliphatischen Resten der Formeln (1a), (1b)

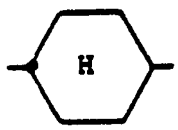
(1a)

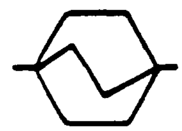
(1b)

und aromatischen Resten der Formeln (1c), (1d), (1e)

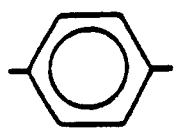
(1c)

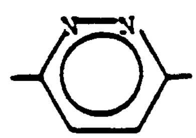
(1d)

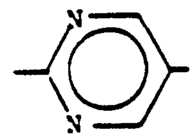
(1e)

X Cyano oder Halogen und Y Sauerstoff, Carboxyl oder Imino ist, $Z^1$ und $Z^2$ gleich oder verschieden sind und Kovalenzbindungen oder Gruppen der Formeln -COO-, $-CH_2O-$ oder

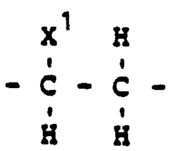

bedeuten, worin $X^1$ das Wasserstoffatom ist oder die für X angegebene Bedeutung hat,
n eine Zahl von 1 bis 12,
und p sowie s jeweils gleich oder verschieden sind und 0 oder 1 bedeuten und R die für $X^1$ angegebene Bedeutung hat oder eine Alkyl-, Alkoxy-, Alkoxycarbonyl-, Alkylcarbonyloxy- oder Alkylaminogruppe, deren Alkylteil jeweils 1 bis 12 C-Atome in gerader oder verzweigter und gegebenenfalls cchiraler Kohlenstoffkette enthält, welche Kette gegebenenfalls einen oder mehrere Substituenten gewählt aus Halogen und Cyano trägt, wobei an jeweils einem Atom der Kette höchstene einer der genannten Substituenten hängt, oder einen Rest der Formel (1f)

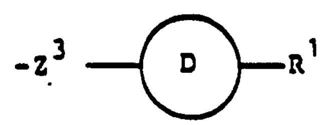 (1f)

bedeutet, in welcher der Ring D die für die Ringe A B und C angegebene Bedeutung, $Z^3$ die für $Z^1$ und $Z^2$ angegebene Bedeutung und $R^1$ die für R angegebene Bedeutung mit Ausnahme des Restes der Formel (1f) hat, mit den Massgaben, dass

(a) an keinen im Molekül vorhandenen cycloaliphatischen Rest der Formeln (1a) oder (1b) gleichzeitig direkt ein Sauerstoffatom einerseits und direkt ein Sauerstoffatom oder Stickstoffatom oder Rest Cyano oder Halogen anderseits gebunden ist,

(b) an keinen im Molekül vorhandenen aromatische Rest der Formel (1c), (1d) oder (1e) Gruppen der Formeln $-CH_2O-$ oder -C(X)(H)- direkt mit den C-Atomen dieser Gruppe gebunden sind ausser wenn X Flour bedeutet,

(c) der Rest $XH_{2n}C_n-$ eine Gruppe der Formel $NC-CH_2CH_2-$bedeutet, falls die Substitution durch X endständig ist,

(d) das Molekül die Formel $NC-CH_2CH_2$ 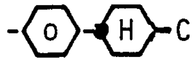

$C_{1-12}$-Alkyl hat, wenn im Molekül der Formel (1) der Ring A einen aromatischen Rest, gebunden an eine $-CH_2CH_2CN$-Gruppe bedeutet, und

(e) $X^1$ nur dann X sein kann wenn R bzw. $R^1$ gleich Halogen oder Cyano ist.

2. Verbindung nach Anspruch 1, dadurch gekennzeichnet, dass der Ring A in Formel (1) einen cycloaliphatischen Rest der Formeln (1a) oder (1b) bedeutet.

3. Verbindung nach Anspruch 1 oder 2, dadurch gekennzeichnet, dass p und s 0 sind.

4. Verbindung nach einem der Ansprüche 1 oder 2, dadurch gekennzeichnet, dass R bzw. $R^1$ einen höchstens zweifach substituierten Alkylteil enthält.

5. Verbindung nach einem der Ansprüche 1-3, dadurch gekennzeichnet, dass R nur dann Cyano, Fluor oder Chlor bedeutet, wenn der direkt mit R verbundene Ring ein aromatischer Rest der Formeln (1c), (1d) oder (1e) ist.

6. Verbindung nach einem der Ansprüche 1-5, dadurch gekennzeichnet, dass die Verbindung der Formel (1) höchstens eine Carboxylgruppe enthält.

7. Verbindung nach einem der Ansprüche 1-5, dadurch gekennzeichnet, dass in der Formel (1) mindestens eines der Brückenglieder $Z^1$, $Z^2$, $Z^3$ die Kovalenzbindung ist.

8. Verbindung nach Anspruch 1, gekennzeichnet durch die Formel (8)

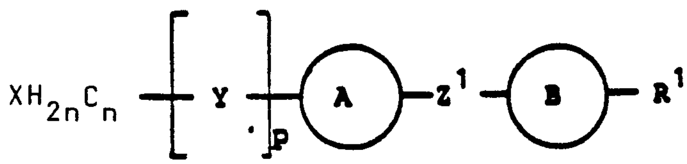

(8)

in der X, Y, A, B, $Z^1$, $R^1$, n und p die in Anspruch 1 angegebene Bedeutung haben.

9. Verbindung nach Anspruch 8, dadurch gekennzeichnet, dass die Ringe A und B Reste der Formeln (1a), (1b) oder/und (1c) bedeuten.

10. Verbindung nach Anspruch 1, gekennzeichnet durch die Formel (9)

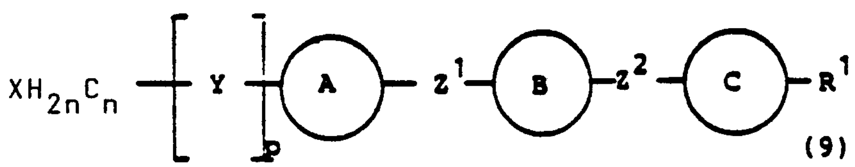

(9)

in der X, Y, A, B, C, $Z^1$, $Z^1$, $R^1$, n und p die in Anspruch 1 angegebene Bedeutung haben.

11. Verbindung nach Anspruch 10, dadurch gekennzeichnet, dass die Ringe A, B und C Reste der Formeln (1a), (1b) oder/und (1c) bedeuten.

12. Verbindung nach einem der Ansprüche 1-11, dadurch gekennzeichnet, dass das C-Atom der Flügelgruppe der Formel $XH_{2n}C_n[Y]_p$, das den Rest X trägt, vom Ring A durch nicht mehr als zwei Atome der Flügelgruppenkette getrennt ist.

13. Verbindung nach einem der Ansprüche 1-12, bei welcher R bzw. $R^1$ einen substituierten Alkylteil enthält, dadurch gekennzeichnet, dass keiner der Substituenten an einem C-Atom des Alkylteils hängt, der vom Ring C bzw. D durch mahr als zwei Kettenatome der Flügelgruppenkette getrennt ist.

14. Flüssigkristallmischung, dadurch gekennzeichnet, dass sie mindestens eine Verbindung gemäß einem der Ansprüche 1-13 enthält.

15. Elektrooptisches Anzeigeelement, dadurch gekennzeichnet dass es eine Flüssigkristallmischung nach Anspruch 14 enthalt.

16. Verwendung einer Verbindung gemäß einem der Ansprüche 1 - 13 als Komponenten von Flüssigkristallmischungen.

**Claims**

1. New anisotropic compounds for use in liquid crystal mixtures for electrooptical displays, characterised by the formula (1)

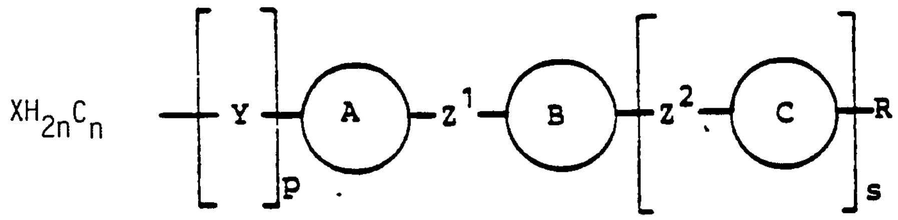 (1)

in which the rings A, B and C are identical or different and are chosen from cycloaliphatic radicals of the formulae (1a) and (1b)

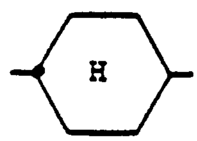

(1a)

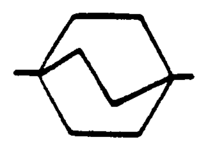

(1b)

and aromatic radicals of the formulae (1c), (1d) and (1e)

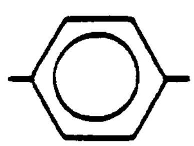

(1c)

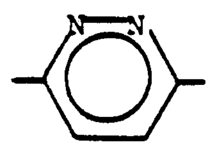

(1d)

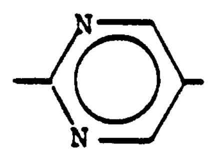

(1e)

X is cyano or halogen

Y is oxygen carboxyl or imino, $Z^1$ and $Z^2$ are identical or different and are covalent bonds or groups of the formula -COO-, $-CH_2O-$ or

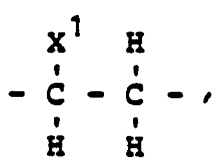,

wherein $X^1$ is the hydrogen atom or has the meaning given for X, n is a number from 1 to 12,

p and s are in each case identical or different and are 0 or 1 and R has the meaning given for $X^1$ or is an alkyl, alkoxy, alkoxycarbonyl, alkylcarbonyloxy or alkylamino group, the alkyl part of which in each case contains 1 to 12 C atoms in a straight or branched, optionally chiral carbon chain, which chain optionally carries one or more substituents chosen from halogen and cyano, at most one of the substituents mentioned in each case being attached to one atom of the chain, or is a radical of the formula (1f)

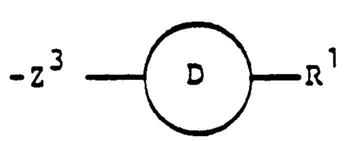 (1f)

in which the ring D has the meaning given for rings A, B and C, $Z^3$ has the meaning given for $Z^1$ and $Z^2$ and $R^1$ has the meaning given for R, with the exception of the radical of the formula (1f), with the provisos that

(a) no oxygen atom, on the one hand, and no oxygen or nitrogen atom or radical cyano or halogen, on the other hand, are at the same time bonded directly to any of the cycloaliphatic radicals of the formulae (1a) and (1b) present in the molecule,

(b) no groups of the formulae $-CH_2O-$ and -C(X)(H)- are bonded directly, with the C atoms of this group, to any of the aromatic radicals of the formulae (1c), (1d) and (1e) present in the molecule, except when X is the fluorine atom,

(c) the radical $XH_{2n}C_n-$ is a group of formula $NC-CH_2CH_2-$ if the substituent X is in terminal position,

(d) the molecule has the formula NC-$CH_2CH_2$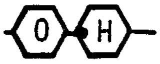

$C_{1-12}$-alkyl if the ring A in the molecule of the formula (1) is a aromatic radical linked to a group -$CH_2CH_2CN$, and

(e) $X^1$ can only be X if R and/or $R^1$ is halogen or cyano.

2. Compound according to Claim 1, characterised in that the ring A in formula (1) is a cycloaliphatic radical of the formula (1a) or (1b).

3. Compound according to Claim 1 or 2, characterised in that p and s are 0.

4. Compound according to one of Claims 1 or 2, characterised in that R and/or $R^1$ contains an alkyl part which is at most disubstituted.

5. Compound according to one of Claims 1-3, characterised in that R only denotes cyano, fluorine or chlorine if the ring bonded directly to R is an aromatic radical of the formula (1c), (1d) or (1e).

6. Compound according to one of Claims 1-5, characterised in that the compound of the formula (1) contains at most one carboxyl group.

7. Compound according to one of Claims 1-5, characterised in that in formula (1) at least one of the bridge members $Z^1$, $Z^2$ or $Z^3$ is a covalent bond.

8. Compound according to Claim 1, characterised by the formula (8)

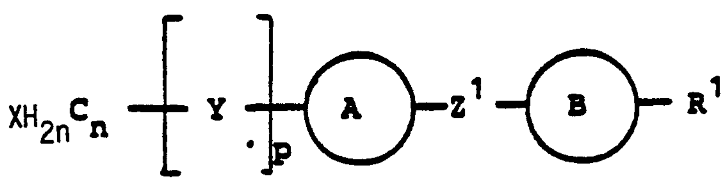

(8)

in which X, Y, A, B, $Z^1$, $R^1$, n and p have the meaning given in Claim 1.

9. Compound according to Claim 8, characterised in that the rings A and B are radicals of the formulae (1a), (1b) or/and (1c)

10. Compound according to Claim 1, characterised by the formula (9)

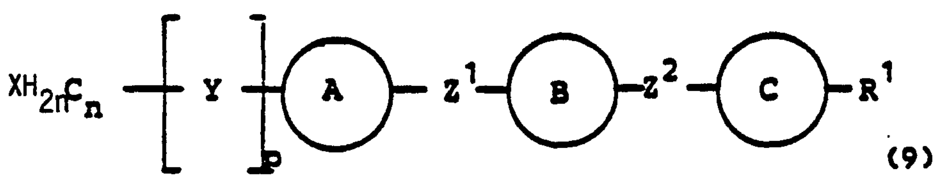

(9)

in which X, Y, A, B, C, $Z^1$, $Z^2$, $R^1$, n and p have the meaning given in Claim 1.

11. Compound according to Claim 10, characterised in that the rings A, B and C denote radicals of the formulae (1a), (1b) or/and (1c).

12. Compound according to one of Claims 1-11, characterised in that the C atom of the end group of the formula $XH_{2n}C_n[Y]_p$, which carries the radical X, is separated from the ring A by not more than two atoms of the end group chain.

13. Compound according to one of Claims 1-12, in which R or $R^1$ contains a substituted alkyl part, characterised in that none of the substituents is attached to a C atom of the alkyl part which is separated from the ring C or D by more than two chain atoms of the end group chain.

14. Liquid crystal mixture, characterised in that it contains at least one compound according to one of Claims 1-13.

15. Electrooptical display element, characterised in that it contains a liquid crystal mixture according to Claim 14.

16. Use of a compound according to one of Claims 1 - 13 as components of liquid crystal mixtures.

**Revendications**

1. Nouveaux composés anisotropes pour l'utilisation dans des mélanges à cristaux liquides servant dans des affichages électro-optiques, caractérisés par la formule 1

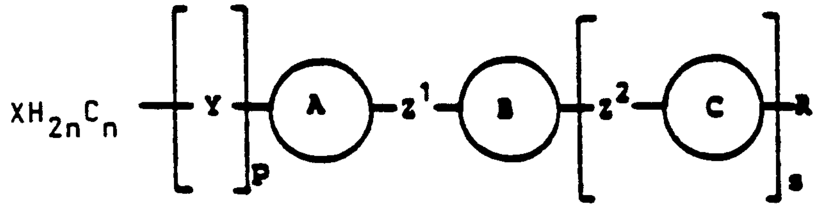 (1)

dans laquelle les cycles A, B et C, identiques ou différents, sont choisis parmi les groupes cycloaliphatiques de formules 1a, 1b

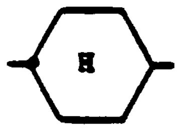

(1a)

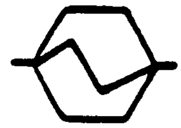

(1b)

et les groupes aromatiques de formules 1c, 1d, 1e

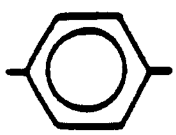

(1c)

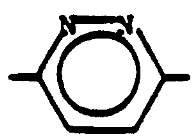

(1d)

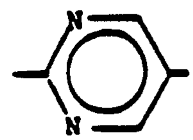

(1e)

X représente un groupe cyano ou un halogène et Y représente l'oxygène, un groupe carboxyle ou imino,

$Z^1$ et $Z^2$, ayant des significations identiques ou différentes, représentent des liaisons covalentes ou des groupes de formules -COO-, $-CH_2O-$ ou

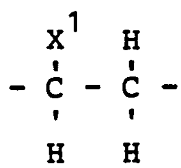

dans lequel $X^1$ représente l'atome d'hydrogène ou a les significations indiquées pour X,

n est un nombre de 1 à 12, et

p et s, ayant des significations identiques ou différentes, sont chacun égaux à 0 ou 1 et R a les significations indiquées pour $X^1$ ou représente un groupe alkyle, alcoxy, alcoxycarbonyle, alkylcarbonyloxy ou alkylamino dont la partie alkyle contient dans chaque cas 1 à 12 atomes de carbone en chaîne droite ou ramifiée et le cas échéant chirale, ladite chaîne portant le cas échéant un ou plusieurs substituants choisis parmi les halogènes et les groupes cyano, étant spécifié qu'un atome de la chaîne porte au maximum un des substituants mentionnés, ou un groupe de formule 1f

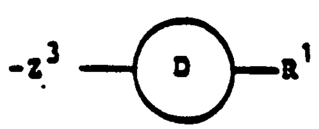 (1f)

dans laquelle le cycle D a les significations indiquées pour les cycles A, B et C, $Z^3$ a les significations indiquées pour $Z^1$ et $Z^2$ et $R^1$ les significations indiquées pour R à l'exception du groupe de formule 1f, avec les restrictions suivantes:

(a) sur aucun des groupes cycloaliphatiques de formule 1a ou 1b existant dans la molécule, il ne peut y avoir simultanément liaison directe d'un atome d'oxygène d'une part et liaison directe d'un atome d'oxygène ou d'un atome d'azote ou du groupe cyano ou d'un halogène d'autre part,

(b) sur aucun des groupes aromatiques de formule 1c, 1d ou 1e existant dans la molécule, il ne peut y avoir liaison directe de groupes de formule $-CH_2O-$ ou -C(X) (H)- par l'intermédiaire des atomes de carbone de ce groupe, sauf lorsque X représente le fluor,

(c) le groupe $XH_{2n}C_n$- est un groupe de formule NC-$CH_2CH_2$- lorsque la substitution par X est en position terminale,

(d) la molécule répond à la formule NC-$CH_2CH_2$-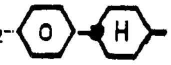

$C_{1-12}$-Alkyl lorsque, dans la molécule de formule 1, le cycle A est un groupe aromatique relié à un groupe -$CH_2CH_2CN$, et

(e) $X^1$ ne peut représenter que X lorsque R ou $R^1$ représente un halogène ou le groupe cyano.

2. Composé selon la revendication 1, caractérisé en ce que le cycle A de la formule 1 est un groupe cycloaliphatique de formule 1a ou 1b.

3. Composé selon la revendication 1 ou 2, caractérisé en ce que p et s sont égaux à 0.

4. Composé selon l'une des revendications 1 ou 2, caractérisé en ce que R et $R^1$ contiennent une partie alkyle substituée deux fois au maximum.

5. Composé selon l'une des revendications 1 à 3, caractérisé en ce que R ne peut représenter qu'un groupe cyano, le fluor ou le chlore lorsque le cycle relié directement à R est un groupe aromatique de formule 1c, 1d ou 1e.

6. Composé selon l'une des revendications 1 à 5, caractérisé en ce que le composé de formule 1 contient au maximum un groupe carboxyle.

7. Composé selon l'une des revendications 1 à 5, caractérisé en ce que, dans la formule 1, au moins un des ponts $Z^1$, $Z^2$, $Z^3$ est une liaison covalente.

8. Composé selon la revendication 1, caractérisé par la formule 8

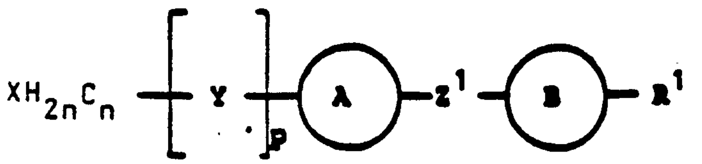
(8)

dans laquelle X, Y, A, B, $Z^1$, $R^1$, n et p ont les significations indiquées dans la revendication 1.

9. Composé selon la revendication 8, caractérisé en ce que les cycles A et B sont des groupes de formule 1a, 1b et/ou 1c.

10. Composé selon la revendication 1, caractérisé par la formule 9

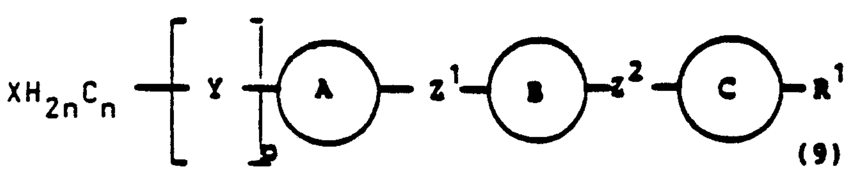
(9)

dans laquelle X, Y, A, B, C, $Z^1$, $Z^2$, $R^1$ n et p ont les significations indiquées dans la revendication 1.

11. Composé selon la revendication 10, caractérisé en ce que les cycles A, B et C sont des groupes de formules 1a, 1b et/ou 1c.

12. Composé selon l'une des revendications 1 à 11, caractérisé en ce que l'atome de carbone du groupe d'extrémité de formule $XH_{2n}C_n[Y]_p$, qui porte le reste X, est séparé du cycle A par deux atomes au maximum de la chaîne du groupe d'extrémité.

13. Composé selon l'une des revendications 1 à 12, dans lequel R ou $R^1$ contient une partie alkyle substituée, caractérisé en ce qu'aucun des substituants n'est placé sur un atome de carbone de la partie alkyle qui est séparée du cycle C ou D respectivement par plus de deux atomes de chaîne de la chaîne du groupe d'extrémité.

14. Mélange à cristaux liquides, caractérisé en ce qu'il contient au moins un composé selon l'une des revendications 1 à 13.

15. Elément d'affichage électro-optique, caractérisé en ce qu'il contient un mélange à cristaux liquides selon la revendication 14.

16. Utilisation d'un composé selon l'une des revendications 1 à 13 en tant que composant de mélanges à cristaux liquides.